# EUROPEAN PATENT APPLICATION

(11) **EP 1 808 149 A1**
(43) Date of publication of application: **18.07.2007**
(21) Application number: 07250074.7
(22) Date of filing: 09.01.2007
(51) Int. Cl.: A61F 2/06

(54) **Intraluminal stent graft**

(30) Priority: 11.01.2006 US 329567
(71) Applicant: Cordis Corporation, Miami Lakes, FL 33014 (US)
(72) Inventor: Davila, Luis, Alpharetta GA 30005 (US); Feller, Frederick, III, Coral Springs FL 33076 (US)
(74) Representative: Belcher, Simon James

(57) **Abstract**

A modular intraluminal stent graft. The intraluminaL stent graft is bifurcated having a primary section and a secondary section extending therefrom. The primary section tapers from a larger diameter at an upstream end to a smaller diameter at a downstream end. The downstream end of the primary section has a pair of independent openings each having an expanded diameter. The secondary section provides a first endoleg having an upstream end that is received through the expanded diameter of one opening of the primary section, and a second endoleg having an upstream end that is received through the second opening of the primary section. The upstream ends of each endoleg, in its expanded state, is larger than the downstream portion of the respective endolegs and expands within the primary section to help assemble the graft *in situ.* The first and second endolegs also expand within the respective openings each is received within to assemble the stent graft *in situ* as well. The primary section is positioned within a blood vessel trunk, whereas the endolegs of the secondary section are positioned within a blood vessel branched from the blood vessel trunk. A typical application would be to place the primary section within the abdominal aorta infrarenally, with the endolegs positioned in the ipsilateral and contralaterial iliacs, respectively, By minimizing the number of stent segments in the primary section of the stent graft a lower delivery profile is achieved.

## Description

The invention generally relates to an intraluminal stent graft. More specifically, the invention relates to a bifurcated stent graft assembled *in situ* for treating vascular aneurysms.

Stents are commonly used to repair compromised blood vessels in the body. Such stcnts may be used to repair compromised coronary arteries which have become narrowed or altogether blocked by the build up of plaque. They may also be used to replace compromised blood vessels, such as the aorta, which have developed enlarged, weakened areas known as aneurysms. In the aorta, aneuryms may often occur in the areas where the aorta divides into two secondary arteries, such as the two common iliac arteries, which supply blood to the lower limbs.

In the past, abdominal aortic aneurysms were frequently repaired by surgery. Such surgery typically required an incision in the patient's body, cutting into the aorta, and implanting a tubular graft into the artery to replace the portion of the artery compromised by the aneurysm. Such surgery poses obvious risks, extended hospital stays, and relatively long recovery periods of up to many months.

Intraluminal stent grafts have been developed as an alternative to surgery. In the case of abdominal aortic aneurysms, such intraluminal stent grafts are often bifurcated stent grafts having a primary section anchored within the abdominal aortic artery at or above the bifurcation of the artery, and at least two secondary sections each of which extends from the primary section and into the arterial sections, such as the ipsilateral and contralateral iliacs, branching from the abdominal aortic artery.

Many conventional stent grafts include stent segments in the primary section of the stent graft. The inclusion of stent segments tends to limit the profile reduction of the stent graft however. Larger delivery systems, i.e., greater than 15F, for example, are thus often required to delivery conventional stent grafts to intended treatment sites, and thus require a surgical cut down of the groin to obtain vascular access. Moreover, conventional stent grafts are susceptible to endoleaks, i.e., Type I and III, stent graft disconnection, or migration, particularly where the stent grafts is modular or secured in place by radial forces upon expansion of the stent graft.

In view of the above, a need exists for a modular bifurcated stent graft formed *in situ* that provides a low delivery profile for percutaneous delivery and minimizes endoleaks and migrations when emplaced at an intended treatment site.

The invention comprises a modular intraluminal stent graft assembled *in situ* for treating various conditions in the vasculature of a patient. The modular intraluminal stent graft minimizes the delivery profile of the stent graft and delivery catheter used therefore, and minimizes endoleaks and migrations of the stent graft after emplacement thereof at an intended treatment site. The expandable materials comprising the radially expandable stent segments described herein are preferably self expanding materials, such as Nitinol, or other such self-expanding materials known in the art, although balloon expandable materials may also be used as the artisan should readily appreciate.

The modular intraluminal stent graft comprises an expandable primary section and an expandable secondary section. Each of the primary section and the secondary section expands from a crimped state, for delivery thereof to an intended treatment site, to an expanded state when deployed. Ideally, in the crimped state, the modular intraluminal stent graft minimizes a delivery profile thereof to enable delivery via a 12F-15F delivery catheter system, for example. Minimizing the number of radially expandable stent segments within the primary section helps to achieve the lower profiled delivery status of the modular intraluminal stent graft and enables the use of the smaller profiled delivery catheter as a result.

The primary section of the modular intraluminal stent graft further comprises an upstream end and a downstream end, The upstream end has a larger expanded diameter than the downstream end. The downstream end further comprises a first opening and a second opening. In practice, the primary section is generally positioned within a blood vessel trunk, such as infrarenally within the abdominal aorta, and endolegs of the secondary section are positioned within blood vessels branching from the blood vessel trunk, such as within the ipsilaterial and contralateral iliacs, for example. A region between the upstream and downstream ends of the primary section preferably omits stent segments to enable the lower delivery profile of the stent graft.

The secondary section comprises a first endoleg and a second endoleg, wherein the first endoleg is received *in situ* through the first opening of the primary section and the second endoleg is received *in situ* through the second opening of the primary section such that an upstream end of each endoleg flares to its expanded state within the region of the primary section otherwise omitting stent segmentsThe flared upstream end of each endoleg comprises radially expanding stent segments that the primary section region otherwise omits. The flared upstream end of each endoleg thus helps to seal and anchor the endoleg within a respective opening of the primary section. Graft material covers the primary and secondary sections. One of the endolegs is thus positioned within one blood vessel branch, whereas the other endoleg is positioned within another blood vessel branch.

The upstream end of the primary section further comprises a series of radially expandable stent segments. The primary section preferably further comprises a single band of radially expandable stent segments within each of the first and second openings at the downstream end of the primary section. Preferably, the single band of radially expandable stent segments in one of the first and second openings is offset from the single band of radially expandable stent segments in the other of the first and second openings. Longitudinal ribs may be provided in the primary section region between the series of stent segments in the upstream end of the primary section and the single bands of radially expandable stent segments in the respective openings at the downstream end of the primary section.

The primary section may further comprise barbs or hooks to help fixate the primary section within the blood vessel at the intended treatment site, such as, for example, the infrarenal aortic neck, or superior to the renal arteries. Downstream ends of one or both of the endolegs may further comprise an expandable section to help anchor the one or both endolegs in place within a blood vessel branch in which the respective endoleg is received, for example, such as to help anchor the one or both endolegs within the ipsilateral or contralateral iliacs branched from the abdominal aorta. Other agents, such as foams, textiles or films may be incorporated onto either or both of the primary section or secondary section to effect an even better seal between the stent graft segments and vasculature, and/or between the primary and secondary sections if desired.

The present invention can be used in a method for delivering an intraluminal stent graft to an intended treatment site, the method comprising:
delivering a primary section of the intraluminal stent graft within a vessel trunk via a catheter;
expanding upstream and downstream ends of the primary section within the vessel trunk by withdrawal of the catheter, wherein the downstream end of the primary section comprises first and second openings;
delivering a first endoleg through one of the first and second openings of the primary section via a catheter;
assembling the first endoleg *in situ* by expanding the upstream end of the first endoleg within the primary section, a portion of a downstream end of the first endoleg in the first opening of the primary section, and a remaining portion of the downstream end of the first endoleg in a first blood vessel branch ;
delivering a second endoleg into the primary section through the second opening of the primary section via a separate catheter, the second endoleg having an upstream end and a downstream end; and
assembling the second endoleg *in situ* by expanding the upstream end of the second endoleg within the primary section, a portion of the downstream end of the second endoleg within the second opening of the primary section, and a remaining portion of the second endoleg in another blood vessel branch, the expanded upstream ends of the first and second endolegs each having at least one stent segment that interfacing with a region of the primary section that otherwise omits stent segments.

The primary section and the first endoleg may be delivered using the same catheter. Alternatively, the catheter delivering the primary section and the catheter delivering the first endoleg may be different.

At least one stent segment in each of the upstream ends of each endoleg may expand to fill a region of the primary section that otherwise omits stent segments.

The method may further comprise providing barbs at the upstream end of the primary section and anchoring the barbs within the blood vessel trunk.

The blood vessel trunk may be an abdominal aorta, the first blood vessel branch may be an ipsilateral iliac, and the second blood vessel branch may be a contralateral iliac,

The method may also further comprise deploying an expandable suprarenal fixation stent in the abdominal aorta with the primary section.

The method may also further comprise expanding downstream ends of at least one of the first and second endolegs to anchor within the respective blood vessel branch the endoleg is deployed within.

The graft material may surround the primary and secondary sections, and not the suprarenal fixation stent.

Embodiments of the invention will now be described by way of example with reference to the accompanying drawings, in which:
Figure 1 illustrates an assembled view of a modular intraluminal stent graft as described herein.
Figure 2 illustrates a view of a primary section of the modular intraluminal stent graft of Fig. 1.
Figure 3 illustrates a view of a secondary section of the modular intraluminal stent of Fig. 1.
Figures 4-8 illustrate various steps of deploying a modular intraluminal stent graft as described herein.
Figure 9 illustrates a variation of a modular intraluminal stent graft with suprarenal fixation capacities as described herein,

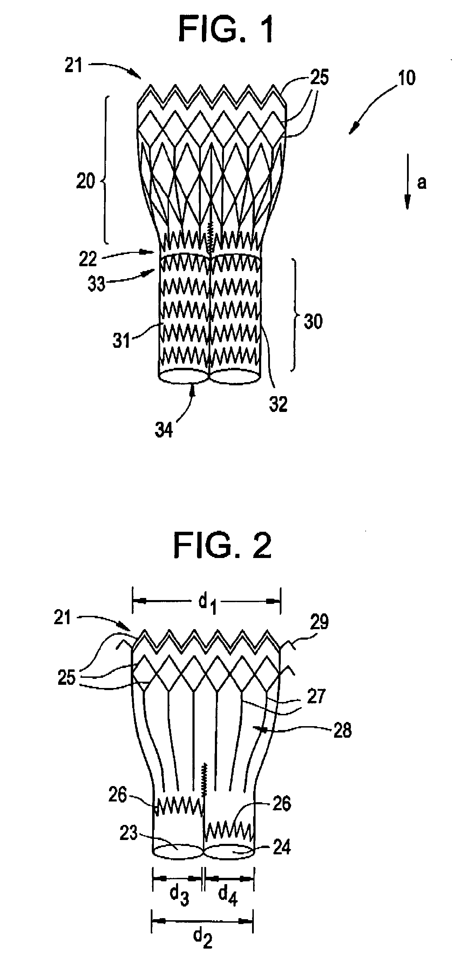

Fig. 1 illustrates an assembled view of an embodiment of a modular intraluminal stent graft 10 according to the description herein. The modular intraluminal stent graft 10 comprises an expandable primary section 20 and an expandable secondary section 30. Each of the primary section 20 and the secondary section 30 expands from a crimped state, for delivery thereof to an intended treatment site, to an expanded state when deployed. The expanded state is shown in Fig. 1. In the crimped state, the modular intraluminal stent graft 10 ideally minimizes a delivery profile thereof to enable delivery via a conventional 12F-15F delivery catheter system, for example. Minimizing the number of radially expandable stent segments within the primary section 20 helps to achieve the lower profiled delivery status of the modular intraluminal stent graft 10. The materials used to comprise the expandable portions of the intraluminal stent graft 10 described herein are preferably self-expandable materials, such as Nitinol, or other self-expandable materials known in the art, although the expandable portions may instead be comprised of balloon expandable materials, as also known in the art.

Referring now to Figs. 1 and 2, the primary section 20 of the modular intraluminal stent graft 10 further comprises an upstream end 21 and a downstream end 22, wherein "upstream" is understood as opposite the direction of blood flow (arrow a), and "downstream" is understood as in the same direction as blood flow (a). In the expanded state, the upstream end 21 comprises a first diameter d₁ that is larger than a second diameter d₂ at the downstream end 22 of the primary section 20. As a result, the primary section 20 tapers from the larger first diameter d₁ to the smaller second diameter d₂.

The downstream end 22 of the primary section 22 is further comprised of a first opening 23 and a second opening 24. The second diameter d₂ is thus comprised of the third diameter d₃ of the first opening 23, and the fourth diameter d₄ of the second opening 24. The expanded diameter d₃ and d₄ of the respective first and second openings 23, 24 is each configured to receive a respective upstream end 33 of first and second endolegs 31, 32 of the secondary section 30. The third diameter d₃ and the fourth diameter d₄ of the respective openings 23, 24 may be the same, or different, relative to one another. In practice, the primary section 20 is deployed within a blood vessel trunk, such as the abdominal aorta AA, whereas the endolegs 31, 32 of the secondary section are deployed within blood vessels that branch off from the trunk, such as the ipsilateral iliac IA and contralateral iliac CA for addressing an abdominal aortic aneurysm AAA, for example (Fig.s. 4-8), although other trunk and branched vessels arrangements may also be conducive to use of the stent graft and methods described herein. Although shown and described herein as an intraluminal stent graft 10 having a primary section deployable within the abdominal aorta AA and endolegs 31, 32 of a secondary section deployable within an ipsilateral artery IA and a contralateral artery CA, respectively, for treating an abdominal aortic aneurysm AAA, the artisan will readily appreciate that the stent graft 10 may be deployed for treating other vascular aneurysms or conditions. Moreover, even when deploying the stent graft 10 for treatment of an abdominal aortic aneurysm AAA, the endolegs 31, 32 may be deployed in the downstream openings of the primary section or in either of the ipsilateral or contralateral arteries, other than as shown and described herein, in the medical practitioner's discretion.

Referring again to Figs. 1 and 2, the upstream end 21 of the primary section 20 further comprises a series of radially expandable stent segments 25. The primary section 20 preferably further comprises a single band of radially expandable stent segments 26 within each of the first and second openings 23, 24 at the downstream end 22 of the primary section 20. Preferably, the single band of radially expandable stent segments 26 in one of the first and second openings is offset from the single band of radially expandable stent segments in the other of the first and second openings. A region between the series of stent segments 25 in the upstream end 21 of the primary section and the single bands of radially expandable stent segments 26 in the respective openings 23, 24 at the downstream end 22 of the primary section 20 preferably omits stent segments as otherwise exist in the upstream or downstream ends of the primary section. Instead, longitudinal ribs 27 may be provided in the region between the series of stent segments 25 at the upstream end 21 and the single bands of stent segments 26 at the downstream end 22 of the primary section 20. When fully deployed within the vessel trunk, such as within the abdominal aorta AA, the radially expanding stent segments 35 provided in upstream ends 33 of the first and second endolegs 31, 32 flare to their expanded states within the primary section 20 and help seal and anchor the endolegs 31, 32 within the respective opening 23, 24 within which the endolegs are received. The radially expanding stent segments 35 of the upstream ends 33 of the endolegs 31, 32 thus further expand and hold open the graft material 28 notwithstanding the omission of such stent segments in the region between the upstream and downstream ends 21, 22 of the primary section 20. By minimizing the number of stent segments in the primary section 20 in this manner, and by off-setting the single bands of stent segments 26 in the respective openings 23, 24 of the downstream end 22 of the primary section 20, the stent graft 10 is more readily crimped to a lower delivery profile-Graft material 28 surrounds the primary section 20.

The radially expandable stent segments 25 within the upstream end 21 of the primary section 20 help to anchor the primary section 20 within the blood vessel trunk, whereas the preferably single band of radially expandable stent segments 26 around each of the first and second openings 23, 24 at the downstream end 22 of the primary section 20 help to maintain the respective openings 23, 24 open during cannulation and *in situ* assembly of the respective endolegs 31, 32 of the secondary section 30 through the openings 23, 24 of the primary section 20.

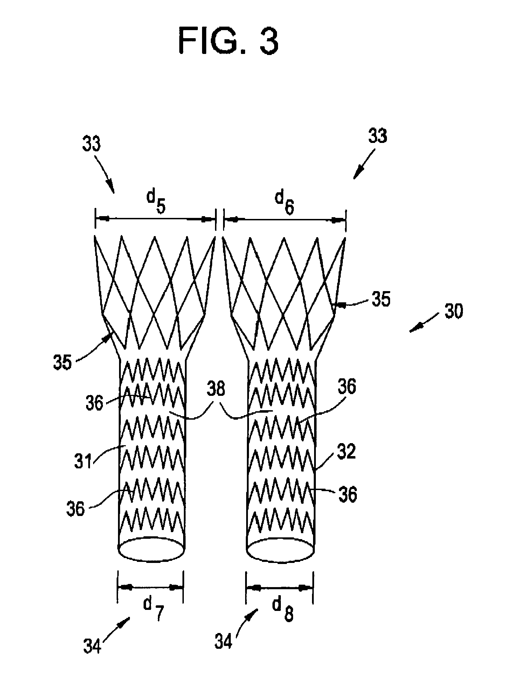

Fig. 3 illustrates the secondary section 30 in greater detail, wherein Figs. 1-3 are referred to hereinbelow, The secondary section 30 of the modular intraluminal stent graft 10 further comprises a first expandable endoleg 31 and a second expandable endoleg 32. Each endoleg 31, 32 comprises an upstream end 33, and a downstream end 34. The upstream end 33 of each endoleg 31, 32 is further comprised of at least one radially expandable stent segment 35 that, in its expanded state, flares to a diameter d₅, d₆ larger than the expanded diameter d₃, d₄ of the respective first or second opening 23, 24 of the primary section 20 through which the respective endoleg 31, 32 is received. For example, the upstream end 33 of the first endoleg 31, in its expanded state, comprises a fifth diameter d₅ that flares to larger than the third diameter d₃ of the first opening 23 at the downstream end 22 of the primary section 20, whereas the upstream end 33 of the second endoleg 32, in its expanded state, comprises a sixth diameter d₆ that flares to larger than the fourth diameter d₄ of the second opening 24 at the downstream end 22 of the primary section 20, Ideally, the combination of the upstream end 33 diameters d₅, d₆ of the endolegs 31, 32 in the expanded state combine to fill the region of the primary section 20 between the upstream end 21 and the downstream end 22 of the primary section 20 that is otherwise free of stent segments. In this manner, the stent segments 35 of the upstream ends 33 of the endolegs 31, 32 help to secure the primary section 20 in place within the trunk vessel and help to seal and anchor the endolegs 31, 32 in place within the respective openings 23, 24 of the primary section 20. The downstream end 34 of each endoleg 31, 32 is further comprised of a series of radially expandable stent segments 36 generally exhibiting, in their expanded states, a diameter d₇ or d₈, respectively, equal to, or less than, the expanded third or fourth diameter d₃, d₄ of the openings 23, 24 of the primary section 20 from which the respective endoleg 31, 32 extends. As before, graft material 38 surrounds the secondary section 30.

Sealing of the endolegs 31, 32 of the secondary section 30 within a respective one of the openings 23, 24 of the primary section 20 is thus effected by the expanded diameters d₅, d₆ of the upstream ends 33 of the respective endolegs 31, 32 within the primary section 20 and by expanded diameters d₇, d₈ of an upstream portion of the endolegs 31, 32 within the expanded diameters d₃, d₄ of the respective openings 23, 24 at the downstream end 22 of the primary section 20 of the stent graft 10. Other agents, such as foams, textiles or films may be incorporated onto either or both of the primary section or secondary section to effect an even better seal if desired.

The primary section 20 may further comprise barbs or hooks to help anchor or fixate the primary section 20 within the blood vessel trunk at the intended treatment site. If desired, one or both of the downstream ends 34 of the endolegs 31, 32 may further comprise an expandable end, such as the upstream end 33 thereof, to help anchor the one or both endolegs in place within a blood vessel branch in which the respective endoleg is received. Where provided, the additional expandable end of the endolegs is also preferably comprised of self-expandable materials such as Nitinol, or other known self-expandable materials, or alternatively of balloon expandable materials known in the art.

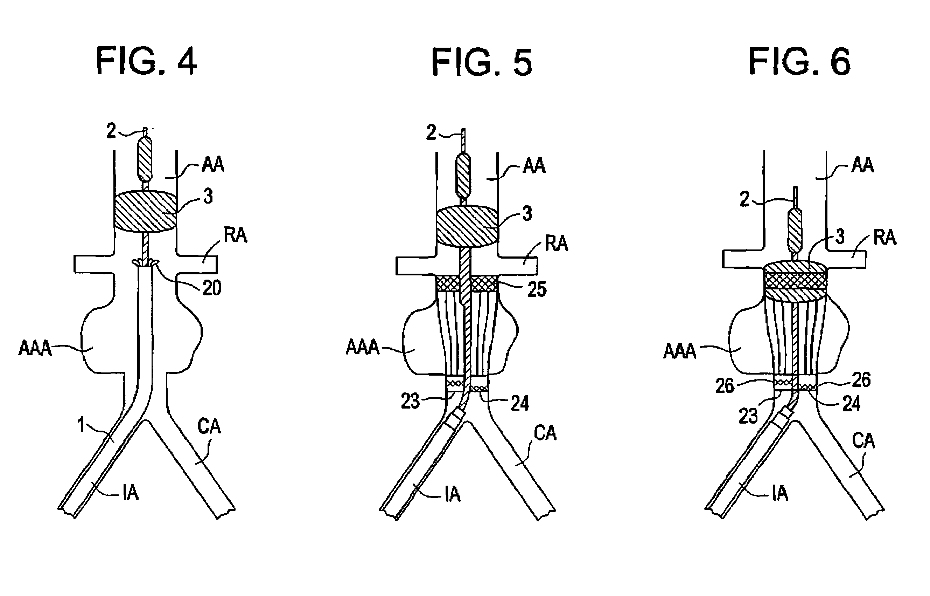

Figs. 4-8 illustrate various steps of delivering a modular intraluminal stent graft 10 to an intended treatment site, such as infrarenally within in an abdominal aorta AA to address an abdominal aortic aneurysm AAA, for example. In Fig. 4, the intraluminal stent graft 10 is mounted in its crimped state onto a conventional low profile delivery catheter 1. The delivery catheter 1 is preferably 12F-15F and includes a guidewire 2 to help locate the catheter 1 and stent graft 10 within the abdominal aorta AA as desired. The catheter 1 is inserted into the patient through the femoral artery in conventional manner, for example, and until the guidewire 2 penetrates into the abdominal aorta AA beyond the site of the aneurysm AAA and beyond the renal arteries RA. Fluoroscopy, x-ray or other conventional visualization technique may be used to identify when the guidewire 2 has reached its intended position beyond the renal arteries RA. The primary section 20 of the stent graft 10 is then deployed by pushing the primary section 20 out of the catheter 1 or by withdrawing the catheter 1 from over the primary section 20 of the stent graft 10. A balloon 3 may be provided in conventional manner to help provide resistance as the catheter 1 is withdrawn to deploy the primary section 20 of the stent graft 10 infrarenally within the abdominal aorta AA and at the aneurysm AAA site. In either case, as shown in Fig. 5, the series of radially expanding stent segments 25 expand against the abdominal aorta AA as the primary section 20 of the stent graft 10 is delivered from the catheter 1. The catheter 1 is then further withdrawn until the first and second openings 23, 24 at the downstream end 22 of the primary section 20 are deployed. The preferably one band of stent segments 26 in each of the openings 23, 24 expand, as shown in Figs. 5 and 6, against the abdominal aorta AA below the aneurysm AAA as the catheter 1 is withdrawn. Thereafter, the balloon 3, if used, is deflated and withdrawn.

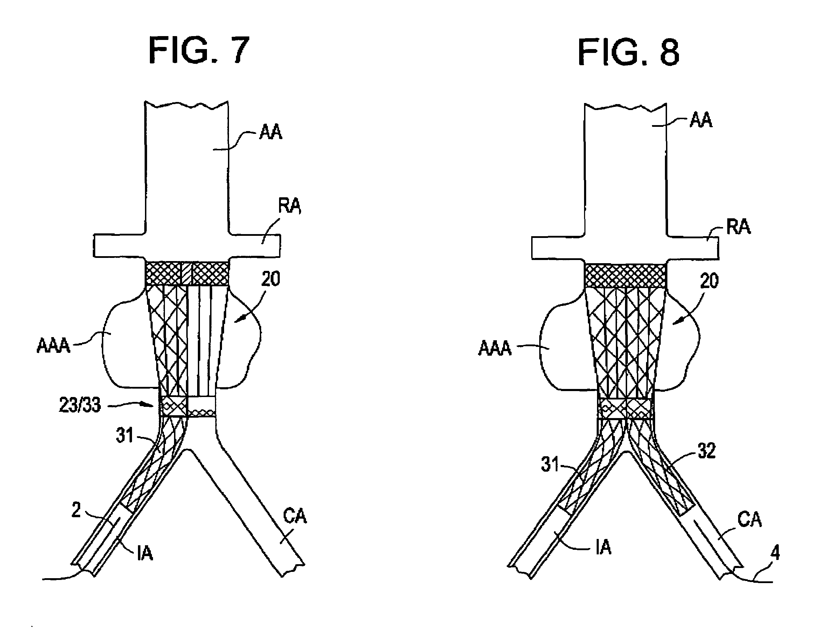

Next, as shown in Fig. 7, the first endoleg 31 is delivered through the femoral artery in conventional manner, for example, until the first endoleg 31 is positioned within the first opening 23 of the primary section 20. The first endoleg 31 may be delivered via the same catheter 1 as delivers the primary section 20, or may be delivered via a separate first endoleg catheter (not shown) after withdrawal of the catheter 1. In either case, fluoroscopy, x-ray or other visualization techniques may be used to help determine the position of the first endoleg 31 as it is delivered. Thereafter, the first endoleg 31 is assembled *in situ* by withdrawing the catheter from which the first endoleg is delivered and radially expanding the least one stent segment 35 at the upstream end 33 of the first endoleg 31 to interface with the region of the primary section 20 that otherwise omits stent segments. As the catheter is further withdrawn, the radially expandable stent segments 36 of the downstream end of the first endoleg 31 expand within the first opening 23 of the primary section 20 and within the blood vessel branch within which the downstream end 34 of the first endoleg 31 is deployed, for example, the ipsilateral iliac IA,. Of course, the downstream end 34 of the first endoleg 31 could instead be deployed within the contralateral iliac CA, for example, in the discretion of the medical practitioner, as the artisan will readily appreciate.

Thereafter, as shown in Fig. 8, the second endoleg 32 is mounted to a second endoleg catheter (not shown) and delivered through the opposite femoral artery in conventional manner, for example, to the intended blood vessel branch similar to as the first endoleg 31 was delivered. Fluoroscopy, x-ray or other visualization techniques may be used to determine the position of the second endoleg 32 during delivery. Once positioned as desired within the second opening 24 of the primary section 20, the second endoleg catheter is withdrawn and the second endoleg 32 is assembled *in situ* within the second opening 24 whereby the at least one radially expandable stent segment 35 at the upstream end 33 of the second endoleg expands and interfaces with with the region of the primary section 20 that otherwise omits stent segments. As the catheter is further withdrawn, the radially expandable stent segments 36 of the downstream end of the second endoleg 32 expand within the second opening 24 of the primary section 20 and within another blood vessel branch, such as the contralateral iliac CA, for example. Of course, where the first endoleg 31 has been deployed within the contralateral iliac CA, then the second endoleg 32 may instead be deployed within the ipsilateral iliac IA, for example, in the discretion of the medical pracitioner, as the artisan will readily appreciate.

If desired, foams, films or other agents may be provided on either or both of the primary and secondary sections to effect an even better seal of the first and second endolegs 31, 32 with the primary section 20 or openings 23, 24 thereof, as discussed above. Likewise, either or both of the endolegs 31, 32 may comprise a further expandable section at a respective downstream end thereof, similar to the radially expandable stent segments 25 at the upstream end 33 thereof, as should be readily appreciated by the artisan. The further expandable sections at the downstream end of either or both endologs 31, 32 would help secure the endolegs 31, 32 in the respective vessel it is deployed within.

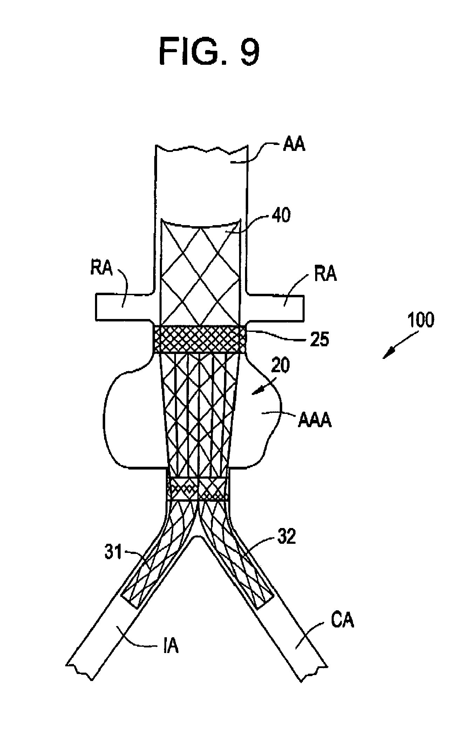

Fig. 9 illustrates a variation of a modular intraluminal stent graft 100 similar to as otherwise described herein, wherein like numerals refer to like components. The intraluminal stent graft 100 of Fig. 9 is generally the same as that shown and described with reference to Figs. 1-8 except that the graft 100 of Fig. 9 further comprises a suprarenal fixation stent 40. The suprarenal fixation stent 40 extends from the upstream end 21 of the primary section 20 beyond the renal arteries RA and affixes to the trunk vessel, for example the abdominal aorta AA, by radial expansion and/or barbs in conventional manner. The endolegs 31, 32 of the secondary section 30 are deployed within the branched vessels, for example the ipsilateral iliac IA and the contralateral iliac CA, as before. The suprarenal fixation stent 40 omits graft material so as not to impede blood flow from the abdominal aorta AA to the renal arteries RA and the kidneys (not shown). The suprarenal fixation stent 40 is preferably comprised of self-expanding materials, such as Nitinol, although it may alternatively be comprised of balloon expandable material.

The various exemplary embodiments of the invention as described hereinabove do not limit different embodiments of the systems and methods of the invention. The material described herein is not limited to the materials, designs or shapes referenced herein for illustrative purposes only, and may comprise various other materials, designs or shapes suitable for the systems and methods described herein, as should be appreciated by the artisan. The various arrangements described herein are illustrative only, and the first and second openings and first and second endolegs may be differently oriented with one another, such as the first opening with the second endoleg or the second opening with the first endoleg, as the artisan should readily appreciate. Moreover, although described with reference to an abdominal aorta vessel trunk and ipsilateral and contralateral iliac vessel branches, such are illustrative only, and other blood vessel configurations are readily contemplated by the modular intraluminal stent and methods described herein.

## Claims

1. A expandable modular intraluminal stent graft comprising:
a primary section that tapers in its expanded state from a larger upstream end diameter to a smaller downstream end diameter;
a series of radially expandable stent segments at the upstream end of the primary section;
a first opening and a second opening, each opening having a diameter in its expanded state that combines to comprise the smaller downstream end diameter of the primary section;
a band of radially expandable stent segment within each opening;
a secondary section comprised of a first endoleg and a second endoleg, each endoleg having an upstream end and a downstream end, the upstream end of each endoleg received within the primary section through a respective one of the openings of the primary section and expanded therein the primary section and the downstream end of the endolegs extending therefrom thre respective one of the openings to assemble the stent graft *in situ;*
a radially expandable stent segment in the upstream end of each endoleg;
a series of radially expandable stent segments in the downstream end of each endoleg; and
graft material surrounding the primary and secondary sections.

2. The modular intraluminal stent graft of claim 1, further comprising:
longitudinal ribs extending between the upstream end and the downsream end of the primary section.

3. The modular intraluminal stent graft of claim 1, wherein the radially expandable stent segment in the respective openings of the primary section are offset relative to one another.

4. The modular intraluminal stent graft of claim 1, wherein the primary section further comprises one or more barbs at the upstream end thereof.

5. The modular intraluminal stent graft of claim 1, wherein one or both of the primary section and the secondary section further comprises one of foams, textiles or films to effect a seal between the primary and secondary sections.

6. The modular intraluminal stent graft of claim 1, further comprising a suprarenal fixation stent extending upstream from the upstream end of the primary section.

7. The modular intraluminal stent graft of claim 1 or 6, wherein the radially expandable stent and/or the suprarenal fixation stent is comprised of self-expanding material.

8. The modular intraluminal stent graft of claim 7, wherein the radially expandable stent and/or the suprarenal fixation stent is comprised of Nitinol.

9. The modular intraluminal stent graft of claim 1 or 6, wherein the radially expandable stent and/or the suprarenal fixation stent is comprised of balloon expandable materials.

10. The modular intraluminal stent graft of claim 1, wherein one or both endolegs further comprises an expandable section at the downstream end thereof that helps anchor the respective endoleg in a vessel.

11. The modular intraluminal stent graft of claim 1, wherein the primary section is configured for deployment within a blood vessel trunk, and each endoleg of the secondary section is configured for deployment within a blood vessel branched from the blood vessel trunk.
